# EUROPEAN PATENT SPECIFICATION

(11) **EP 3 827 803 B1**
(45) Date of publication and mention of the grant of the patent: **07.06.2023**
(21) Application number: 19212055.8
(22) Date of filing: 28.11.2019
(51) Int. Cl.: A61J 15/00, A61M 39/10

(54) **MEDICAL CONNECTOR**
MEDIZINISCHER VERBINDER
RACCORD MÉDICAL

(43) Date of publication of application: 02.06.2021
(73) Proprietor: Axium MTech SA, 1260 Nyon (CH)
(72) Inventor: PICTHALL, Simon, 260 NYON (CH); MILIANI, Marco, 260 NYON (CH); PAZZOLA, Ilaria, 260 NYON (CH); CONFICCONI, Chiara, 260 NYON (CH); BRONZATO, Luca, 260 NYON (CH); LOFARO, Manuele, 260 NYON (CH); NICHETTI, Giuseppe, 260 NYON (CH); DI BARI, Andrea, 260 NYON (CH)
(74) Representative: Caspary, Karsten

(56) References cited:
- US-A1- 2010 185 159
- US-A1- 2015 025 476

## Description

The present invention relates to an improved medical connector, and more particularly to a connector assembly for coupling a tube to a catheter device.

Various situations exist in which a body cavity needs to be catheterized to achieve a desired medical goal. A common situation is to provide nutritional solutions, hydration or medicines directly into the stomach or intestines. For this purpose, during a clinical procedure, a stoma is formed in the stomach or intestinal wall and a catheter or feeding tube is placed through the stoma terminating in the stomach or within the digestive tract. This surgical opening and/or the procedure to create the opening is commonly referred to as "gastrostomy". Feeding solutions can be passed through the catheter to provide nutrients directly to the stomach or digestive tract (known as enteral feeding).

Numerous enteral feeding tubes have been developed over the years, including some having a "low profile" exterior portion which sits flush on the patient's skin commonly referred to as a Low Profile Gastrostomy Tube" or "button", as well as those having the more traditional or non-low profile configurations such as PEG tubes or Gastrostomy Tubes. These enteral feeding tubes are also known as "percutaneous transconduit catheters", "percutaneous transconduit tubes", "gastrostomy catheters", "percutaneous gastrostomy catheters", "PEG catheters" or "enteral feeding catheters". An example of one such device is described in US 6,019,746.

An enteral feeding tube serves as the pathway through the stoma for the transport of feeding solution, hydration or medication into the stomach or digestive tract. During feeding, an enteral feeding tube is often linked to the feeding solution via one or more tubes. In the case of "buttons" these low profile enteral feeding tubes are connected to what is commonly called an "Extension Set" which in turn is connected to what is commonly referred to as an "Administration Set" or "Feeding Set" which itself is then connected to the nutritional solution. In the case of "PEG Tubes" or "Gastro Tubes", no extension set is used and they are directly connected to the "Administration Set".

Because a pressurizing source, e. g. a pump that drives the feeding solution through the tube and into and through the enteral feeding tube, and because feeding may take several hours, e. g. overnight while a patient is sleeping, and in the case of a "Button", maintaining a robust and leak proof connection between the "Extension Set" and the "Button" is an important feature of the connector between these two devices. It is also very desirable that the connection withstand twisting, torquing and pulling forces generated by movement of a patient.

However, a problem of low-profile "Buttons" is the difficulty in connecting and disconnecting the extension sets to and from the "Button" base or head. Many prior art "Button" type enteral feeding tubes have a low-profile base and an indwelling catheter which extends from the base. A distal end of the catheter of such a "Button" device/assembly often includes a balloon which may be expanded within the stomach cavity to hold the Button in position in the stoma.

US 6,019,746 discloses a low profile gastrostomy feeding device which comprises a low-profile cap member having a locking adapter receptacle into which a feeding adapter with a tube connected to it can be inserted and removably secured in a push-and-twist-to-lock fashion. A full rotation of the feeding adapter within the cap member is impossible in this device, and thus the usability is largely reduced.

Some issues of existing connector solutions for the enteral feeding sets have been addressed in EP 2 938 315 A1 wherein a connector assembly for coupling a medical fluid supply tube to the head of a catheter device comprises a circular hub having an annual recess onto which a connector cap with at least two arms may be placed to freely rotate about the circular hub by releasable engagement or snap fit of the arms into the recess. The engagement or snap fit can be effected by manually pressing and releasing wing elements or deflection members on each of the arms. A similar connector assembly is disclosed in US 2013/0296832 A1 and in WO 2015/179094 A1.

These conventional connectors have evident drawbacks that remain unresolved.

When a patient is ready to be fed using such a snap-fit connector, the connector is snap fitted onto the anti-reflux valve assembly by pressing the connector at the two arms or wings in the horizontal direction to urge the circular protrusions of the connector over the circular flange and into the recess located beneath the circular seat. After feeding the snap-fit connector is removed by pressing the two arms or wings again and simultaneously pulling the connector away from the circular hub. The proper attachment of the connector and thus the proper connection of the extension set to the catheter device is thus only effected when both wings or arms are correctly engaged in the recess of the hub. Such correct engagement or snap-fit may not be felt by the user who tries to establish the connection. Due to the lack of tactile feedback the user might have the impression of a correctly established connection which is in fact not properly effected. Further, grasping and correctly applying the necessary horizontal pressure on both wings or arms may not always be an easy task. Thus, the chance of misconnections for the known device is increased.

US 2015/0025476 A1 discloses a low profile gastrostomy feeding tube assembly having a one-way valve and a lock and key mechanism between a locking hub and a compression ring to prevent disconnection of the feeding tube assembly. In one embodiment, when the locking hub and the feeding tube assembly are coupled to one another, a rotation of the feeding tube assembly with respect to the locking hub is enabled.

US 2010/0185159 A1 describes an enteral feeding assembly having a base and a connector, the connector having a key configured to complement a predetermined shape of an opening in the base. Once in position, the connector may rotate 360 degrees relative to the base before being again in the installation/removal position.

Accordingly, it is the object of the present invention to provide a connector assembly which overcomes at least some of the above mentioned disadvantages of the prior art, comprises a simple design, is easy to manufacture and ensures an enhanced performance and usability.

This object is achieved by the connector assembly having the features of claim 1 of the present invention. Advantageous embodiments are described in the subclaims.

According to the invention, there is provided a connector assembly for coupling a tube to a base of a catheter device, the connector assembly comprising a connector element having a channel and being configured to be coupled at a first end to the tube, a revolving lock having an opening for accommodating the connector element and being configured to engage with the connector element thereby enabling a free rotation around a rotation axis, a hub having a tubular channel and being configured to be attached to the base, wherein a second end of the connector element is configured to be at least partially inserted into the tubular channel of the hub, wherein the revolving lock is configured to be releasably locked with the hub, and wherein the rotation of the connector element is configured to be decoupled from the locking mechanism between the revolving lock and the hub wherein the locking mechanism between the revolving lock and the hub comprises at least one thread element on an outer surface of the hub and on an inner surface of the revolving lock. This configuration facilitates a simplified handling while securing a reliable connection between the tube and the catheter device. The decoupling of the rotation of the connector element with respect to the revolving lock from the locking mechanism between the revolving lock and the hub further reduces the chance of an accidental disconnection because turning the connector element will not move the components responsible for the connection and thus not influence the quality of the connection.

Preferably, the locking mechanism comprises a male thread portion on an outer surface of the hub in cooperation with a female thread portion on an inner surface of the revolving lock. Alternatively, there could be a twist-and-lock mechanism without a tilted thread part but with a straight or circular protrusion which engages with a corresponding recess, similar to a bayonet type connection or as in electrical BNC connectors.

Also according to the invention, there is provided a connector assembly for coupling a tube to a base of a catheter device, the connector assembly comprising a connector element having a channel and being configured to be coupled at a first end to the tube, a revolving lock having an opening for accommodating the connector element and being configured to engage with the connector element thereby enabling a free rotation around a rotation axis, a hub having a tubular channel and being configured to be attached to the base, wherein a second end of the connector element is configured to be at least partially inserted into the tubular channel of the hub, wherein the revolving lock is configured to be releasably locked with the hub, and wherein the rotation of the connector element is configured to be decoupled from the locking mechanism between the revolving lock and the hub wherein the locking mechanism between the revolving lock and the hub comprises at least one thread element on an inner surface of the hub and on an outer surface of the revolving lock. Advantageously, the locking mechanism comprises a female thread portion on an inner surface of the hub in cooperation with a male thread portion on an outer surface of the revolving lock. Such thread mechanisms are well known by medical personnel and offer a reliable locking.

It is further beneficial that the locking mechanism comprises at least one protrusion extending from a circumferential surface of the hub in cooperation with at least one recess arranged on a circumferential surface of the revolving lock. Alternatively and similarly it is possible that the locking mechanism comprises at least one protrusion extending from a circumferential surface of the revolving lock in cooperation with at least one recess arranged on a circumferential surface of the hub. This second locking function provides a haptic feedback to the person attaching the revolving lock having the connector element on the hub. Upon engagement of the protrusion with the corresponding recess the user will recognize the completed attachment of the respective components due to the increased force necessary to disengage the two components out of the locking position.

It is also preferred that revolving lock comprises at least one wing element aligned in a plane anywhere from perpendicular or at a slant of up to 45 degrees to the rotation axis. A wing element provides sufficient grip space and enables a user to ensure a proper attachment of the revolving lock on the hub. The wing element may have various shapes, sizes and surface structures configured for a convenient handling by a user.

In a preferred embodiment the connector element comprises an elbow shape wherein the first end is arranged substantially perpendicular to the second end. Such a configuration supports the low profile of the entire catheter assembly including the extension set or the like. It is also possible that the first end of the connector element is not exactly positioned at 90° with respect to the second end; in some cases an angle of between 0° and 90° or between 90° and 150° may be appropriate.

Further, the revolving lock preferably comprises at least one grip element along a circumferential surface. This configuration supports the haptic characteristics of the connector assembly and thus facilitates its handling. The at least one grip element may be a number of parallel grooves or protrusions or a plurality of dimples or notches arranged on the outer surface of the revolving lock. The grip elements need not be evenly distributed along the surface but at predetermined positions to enhance the haptic perception of the connector assembly.

It is also advantageous that the hub comprises interlocking means configured to engage with a corresponding means on the base. This ensures a secure and flawless arrangement of the hub on or in the base making an insertion simple and less error prone. It is particularly important that the interlocking means prevent an unwanted movement or rotation of the hub located on the base in the horizontal direction. Various embodiments are possible for such interlocking means on the corresponding components such as distributed protrusions and recesses which are configured for engagement with each other. Thread portions are also conceivable as well as twist-and-lock elements.

Preferably, the connector element and the revolving lock are preassembled as an integral component. The term "integral component" in this context means that the free rotation of the connector element within the opening of the revolving lock is still ensured at any time after the preassembly. The effect of such a preassembled part is the reduction of components to be handled by the medical personnel when assembling the catheter device and the extension set comprising the connector assembly according to the invention.

It is particularly preferred that the first end of the connector element comprises at least one groove configured to engage with one or more corresponding protrusions in the opening of the revolving lock to form a permanent assembly enabling the free rotation of the connector element with respect to the revolving lock. This configuration leaves sufficient play for a free horizontal rotation of the connector element accommodated within the opening of the revolving lock while keeping both components essentially fixed to each other in the vertical direction. In an alternative embodiment, the arrangement of the groove(s) and protrusion(s) could be the opposite, i.e. the at least one groove could be located on the revolving lock with the corresponding protrusions being located on the first end of the connector element. Such arrangements are not uncommon when using plastic parts in the medical field. The preassembly can be provided by a separate fabrication of both components which are subsequently assembled together. Alternatively, both components could be fabricated as one integral part by 3D-printing.

With further advantage, the first end of the connector element extends beyond the tubular channel of the hub. This ensures a continuous uninterrupted channel from one end of the connector element to the other end which can be directly connected to the corresponding receiving channel in the base. A misconnection can thus be prevented as well as leakages.

Further according to the invention is a catheter assembly comprising a tube, a connector assembly as described above and catheter device having a base.

Preferred embodiments and advantages of the connector assembly according to the invention will now be described with reference to the attached drawings in which:
- Fig. 1: is an exploded perspective view of a first embodiment of the connector assembly according to the invention;
- Fig. 2: is a perspective view of the connector assembly shown in Fig. 1 with all components assembled;
- Fig. 3: is a section view of the embodiment shown in Fig. 2 viewed from a first direction;
- Fig. 4: is a section view of the embodiment in a direction perpendicular to the direction shown in Fig. 3;
- Fig. 5: is a perspective exploded view of the embodiment shown in Fig. 1 viewed from below;
- Fig. 6: is a section view of the connector element of the connector assembly according to the first embodiment shown in Figs. 1 to 5;
- Fig. 7: is a section view of the hub of the catheter assembly according to the first embodiment shown in Figs. 1 to 5;
- Fig. 8: is an exploded perspective view of a connector assembly according to a second embodiment viewed form above;
- Fig. 9: is a perspective exploded view of the connector assembly according to the second embodiment of Fig. 8 viewed from below;
- Fig. 10: is a side view of the connector assembly according to the second embodiment;
- Fig. 11: is a section view of the connector assembly according to the second embodiment; and
- Fig. 12: is an exploded perspective view of a connector assembly according to the first embodiment including a base.

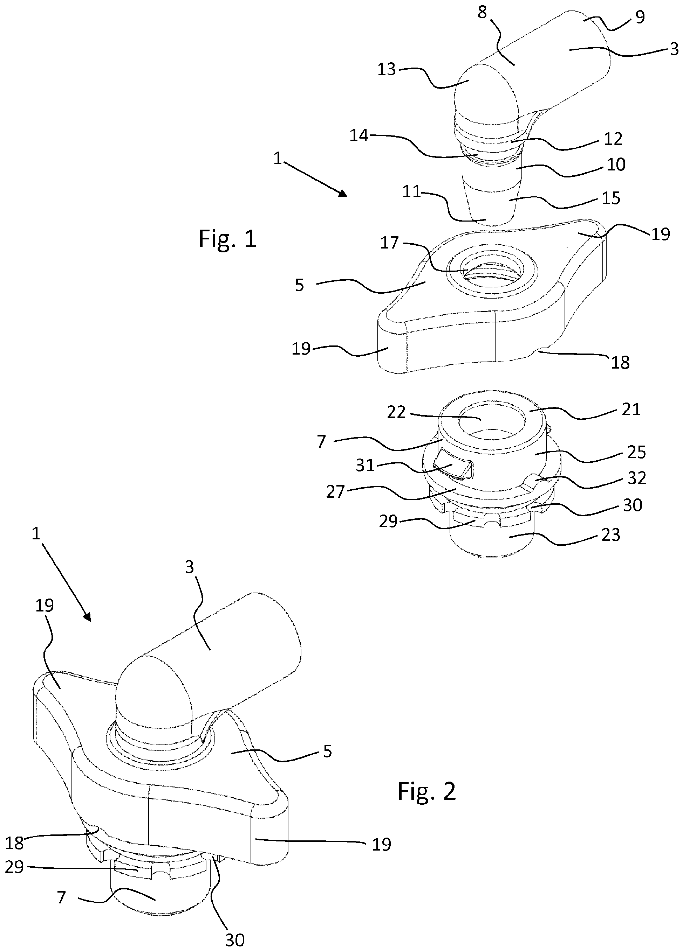

Fig. 1 shows a first preferred embodiment of the connector assembly 1 according to the invention. In this embodiment, connector assembly 1 is essentially comprised of three components: connector element 3, revolving lock 5 and hub 7 which are arranged in series in a vertical direction as can be seen in Fig. 1. Connector element 3 comprises a first tubular portion 8 extending from an elbow junction 13 to a first end 9 in a horizontal direction and a second tubular portion 10 extending from the elbow junction 13 to a second end 11 in a vertical direction. In this first embodiment, first and second tubular portions 8, 10 are arranged essentially perpendicularly with respect to each other, extending from elbow junction 13. However, other angles between 0 degrees and approximately 150 degrees are possible. Connector element 3 includes a channel 6 (not visible in Fig. 1) extending from the first end 9 to the second end 11. First end 9 of connector element 3 is configured to be coupled to a tube, preferably a tube of an extension set in the field of enteral nutrition as will be explained further below. The type of coupling can occur in various ways, e. g. by a press fit of the tube within the inner surface of first end 9.

Second end 11 of connector element 3 comprises a tapered tube portion 15 adjacent to the second cylindrical portion 10 so that second end 11 is insertable into an opening of revolving lock 5 as will be explained below.

In Fig. 1 revolving lock 5 is arranged beneath connector element 3 in such a fashion that the second end 11 of connector element 3 is just above opening 17 on the top surface of revolving lock 5. The general shape of revolving lock 5 is plate-like with two wings 19 extending horizontally opposite to each other which are essentially configured as grip portions in order to improve the handling of the connector assembly 1. The top surface of revolving lock 5 is flat and its height is sufficient to be grasped at wings 19 with the fingers of a user. Opening 17 of revolving lock 5 is essentially circular to accommodate the second cylindrical portion 10 of connector element 3.

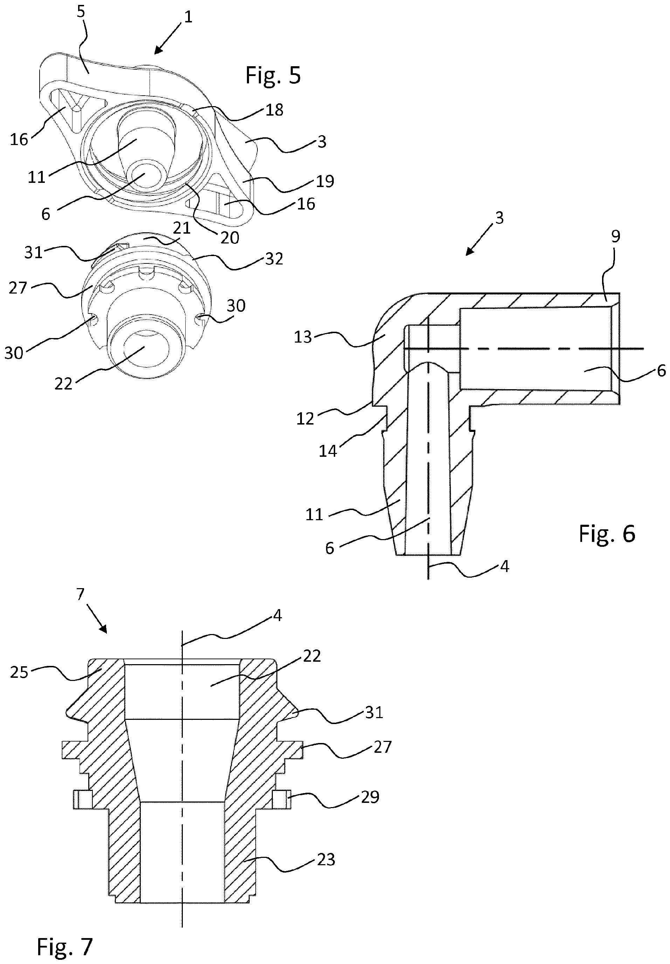

The respective cylindrical portion of connector element 3 and the opening 17 of revolving lock 5 comprise suitable engagement means which are configured to enable a free rotation of connector element 3 with respect to revolving lock 5. These engagement means can be a groove 14 above the second cylindrical portion 10 of connector element 3 and a matching protrusion within opening 17 of revolving lock 5. As will be further explained with respect to Fig. 5 below, connector element 3 and revolving lock 5 may be integrally assembled and delivered/sold as such integral component under the assumption that the free rotation of the connector element 3 within the opening 17 of revolving lock 5 is possible when those two parts are integrally assembled. This usually means that there is a press fit between the corresponding portions on connector element 3 and revolving lock 5 providing sufficient play in the horizontal direction but a positive fit in the vertical direction thus enabling the free rotation of the vertical portion of connector element 3 within opening 17.

Arranged below revolving lock 5 is hub 7 in Fig. 1. Hub 7 extends from upper end 21 to lower end 23 with a tubular channel 22 extending inside hub 7 from top to bottom. A cylindrical outer surface 25 extends from upper end 21 having two threaded portions 31 located opposite to each other. Adjacent to cylindrical outer surface 25 is a first horizontal flange 27 with a protrusion 32 extending vertically from the protruding horizontal surface of first horizontal flange 27. Protrusion 32 is configured to engage with recess 18 in the bottom surface of revolving lock 5 as will be explained in detail below. Below first horizontal flange 27 there is a second horizontal flange 29 on hub 7 having a plurality of radial recesses 30 extending in the horizontal direction and having an essentially semicircular shape in this embodiment. Lower end 23 is a substantially cylindrical portion extending below second horizontal flange 29.

Fig. 2 shows the three components from Fig. 1 assembled together to form connector assembly 1 according to the invention. As already mentioned above, connector element 3 is accommodated within the opening 17 of revolving lock 5 so as to enable free rotation of connecting element 3 with respect to revolving lock 5. Revolving lock 5 in turn is in the depicted embodiment firmly attached to hub 7 wherein a thread portion 20 on the inside surface of revolving lock 5 (not shown in Fig. 2) engages with thread portion 31 on the cylindrical outer surface 25 of hub 7. Furthermore, there is a second locking mechanism which facilitates the attachment of revolving lock 5 with respect to hub 7 and that is the engagement of recess 18 on the lower surface or lower edge of revolving lock 5 engaging with the protrusion 32 on the first horizontal flange 27 of hub 7.

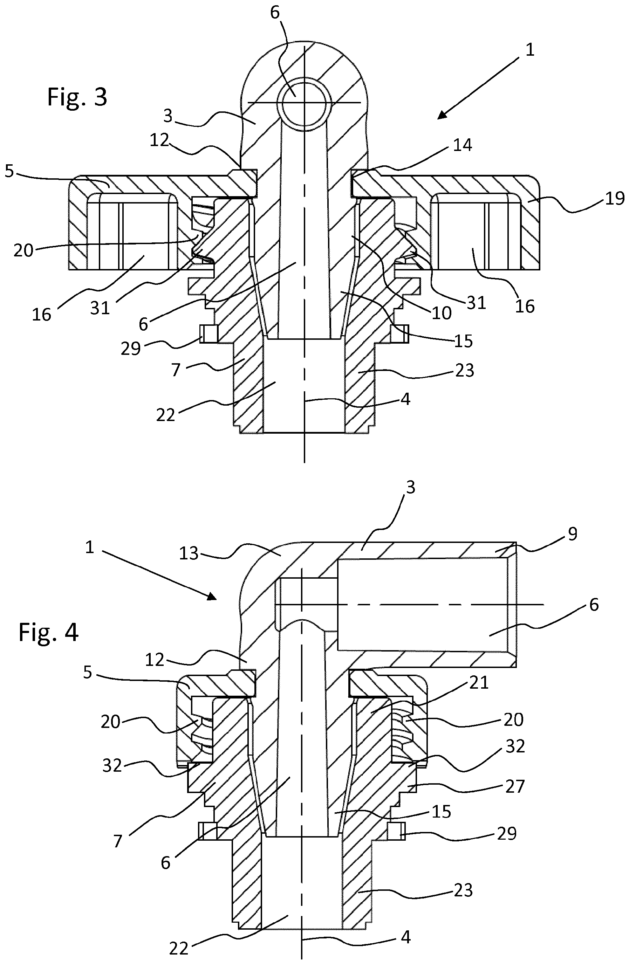

Fig. 3 shows a section view of the connector assembly 1 according to the embodiment depicted in Figs. 1 and 2 in a direction where the revolving lock 5 has its maximum extension in the horizontal direction, i. e. in the drawing plane. As the majority of components have already been described with respect to Figs. 1 and 2, the focus is on those components which have not yet been described in detail. What can be seen in Fig. 3 is the rotation axis which indicates the axis around which the connector element 3 is configured to rotate with respect to the revolving lock 5 and the hub 7. Further, it is noted that the channel 6 within the connector element 3 is of a slightly conical shape which means that it opens up towards the second end 11 of connector element 3. Channel 6 may be otherwise shaped for optimizing fluid flow. On the outside surface of connector element 3 the cylindrical portion 10 and the tapered tube portion 15 each comprise complementarily shaped inner surfaces on hub 7 which are configured such that there is a small gap between the connector element and the hub facilitating the free rotation of this end of connector element 3 within the tubular channel 22 of hub 7.

Fig. 3 also facilitates an enhanced view of the connection between connection element 3 and revolving lock 5. Opening 17 in revolving lock 5 is configured to match with groove 14 in connector element 3 where protrusion 12 forms a type of stop which limits the vertical movement of revolving lock 5 with respect to connector element 3. This engagement or press fit is configured so that connector element 3 can still freely rotate with respect to revolving lock 5 or vice versa, i. e. revolving lock 5 is able to freely rotate around the vertical section of connector element 3 around the rotation axis 4 whereas the vertical movement of revolving lock 5 with respect to connector element 3 is prevented by this engagement mechanism.

The two wing elements 19 on either side of revolving lock 5 include a void 16 which is open from underneath revolving lock 5 in order to save material and thus weight of the component. The central part of revolving lock beneath opening 17 extends further in the horizontal direction and comprises an inner thread portion 20 which is configured to engage with the thread portion 31 on the outside surfaces of hub 7. In Fig. 3 it can be seen how the two thread portions 20, 31 engage with each other to hold revolving lock 5 tight with respect to hub 7 thereby also keeping connector element 3 in a fixed vertical position within tubular channel 22 of hub 7.

Fig. 4 shows connector assembly 1 in the embodiment of Fig. 3 but with a section view in a different viewing angle of about 90 degrees with respect to the view of Fig. 3 so that wings 19 of revolving lock 5 are no longer visible and so that first end 9 of connector element 3 extends to the right hand side in Fig. 4. In the embodiment of Fig. 4 channel 6 of connector element 3 is shown wider at the horizontal portion towards first end 9 than in the central area of elbow junction 13 and than in the vertical portion of connector element 3. Its internal width or diameter increases towards first end 9 to accommodate a matching end of a tube of an extension set.

It is further noted that in Fig. 4 the second locking mechanism between revolving lock 5 and hub 7 can be seen: The edges of first horizontal flange 27 appear thicker in Fig. 4 than in Fig. 3 which is due to the protrusions 32 which in the section view of Fig. 4 add to the overall thickness of the first horizontal flange 27 in the section plane. Thus, the space between the horizontal flange 27 and the underside of revolving lock 5 as shown in Fig. 3 is no longer present in Fig. 4 where it can be seen that the outer surface of revolving lock 5 overlaps with the first horizontal flange of hub 7 due to protrusion 32. This second locking mechanism provides the user of the inventive connector assembly with haptic feedback so as to indicate that revolving lock 5 is now completely and correctly engaged with hub 7, as shown in Fig. 2.

Fig. 5 shows a perspective exploded view of the embodiment shown in figures 1 to 4 wherein the connector element 3 and the revolving lock 5 are preassembled as one integral component with the hub 7 being shown underneath. It can be seen in Fig. 5 that the inner thread portion 20 is arranged all around the inner surface of the underside opening of the revolving lock 5 ready to be engaged with the male thread portion 31 located on the outside surface of hub 7. Further, the two recesses 18 as part of the second locking mechanism between revolving lock 5 and hub 7 are visible as well as the corresponding protrusion 32.

Fig. 6 shows connector element 3 in the embodiment shown in figures 1 to 5. Also here, groove 1 is shown configured to engage with the edge of opening 17 of revolving lock 5. Adjacent to groove 14 is a protrusion functioning as a stop to keep connecting element 3 in place in vertical direction with respect to revolving lock 5. It is again emphasized that there is sufficient play in the horizontal direction between connecting element 3 and revolving lock 5 to enable a free rotation of both components with respect to each other in the horizontal direction.

Fig. 7 shows a section view of hub 7 in the position as in Fig. 3. In this section view male thread portions 31 protrude out of the cylindrical outer surface 25.

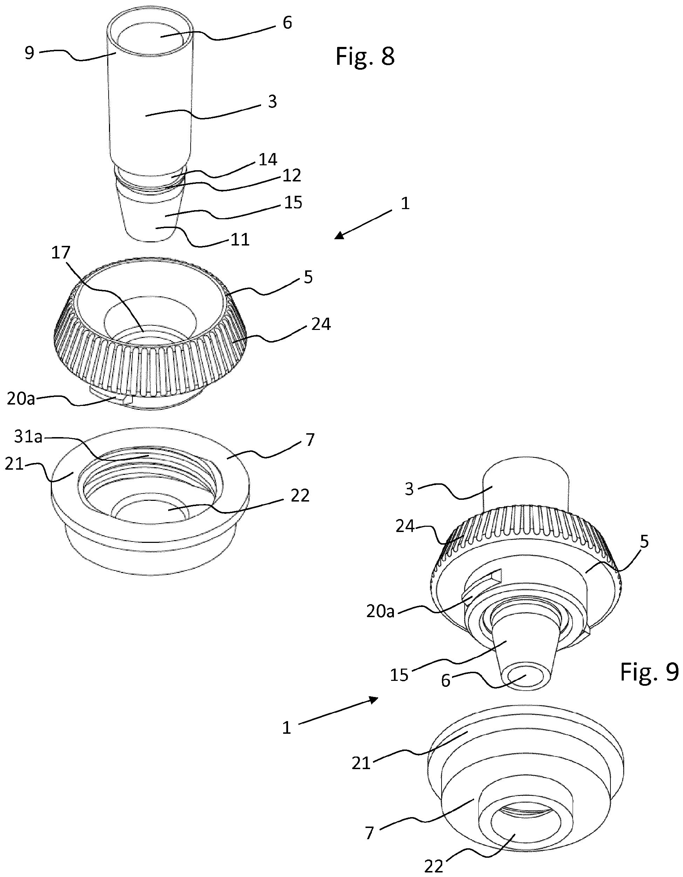

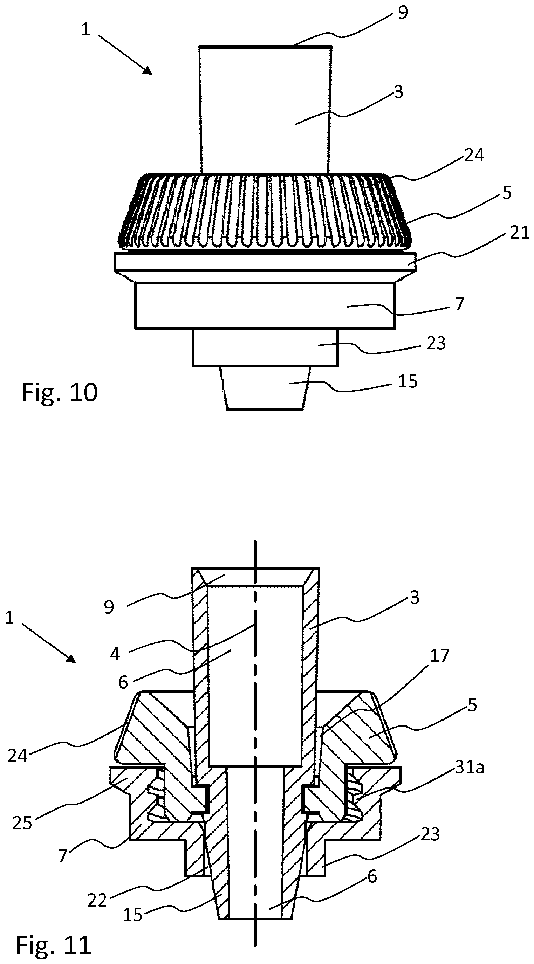

Figs. 8 to 11 show a second embodiment of the connector assembly according to the invention wherein Figs. 8 and 9 show perspective exploded views of the same embodiment and wherein Figs. 10 and 11 show a side view and a section view, respectively, of the second embodiment of the connector assembly according to the invention. Since most of the components of the second embodiment are identical with the ones described with respect to the first embodiment, a description of those components is omitted. The focus of the description of the second embodiment lies in the differences with respect to the first embodiment.

In Fig. 8 it can be seen that the general configuration of the connector element 3 is a longitudinal and no longer a bent configuration. It must be noted, that the connector element can comprise other bend configurations, as described above, not only the version of a 90° angle between the sections extending to the first and the second end, respectively.

Revolving lock 5 of the second embodiment comprises an essentially circular configuration with grip elements 24 arranged on the slanted peripheral outer surface. The configuration of opening 17 is substantially identical to its configuration in this first embodiment. The connection between the connecting element 3 and revolving lock 5 is essentially the same as in the first embodiment: Protrusion 12 and groove 14 of connector element 3 engage with the edge of opening 17 of revolving lock 5 so as to enable a free rotational movement around rotation axis 4 in the horizontal direction but prevent a vertical movement of connector element 3 with respect to revolving lock 5.

The locking mechanism between revolving lock 5 and hub 7 is, however, different from the locking mechanism between those components in the first embodiment. In the second embodiment, the outer surface of the lower portion of revolving lock 5 comprises two male thread portions 20a which are configured to engage with the female thread portion 31a arranged on the entire inner surface of hub 7. It can be seen in Figs. 8 and 9 that the tubular channel 22 of hub 7 is rather short.

Some of the features of the first embodiment have been omitted in the second embodiment such as the second locking mechanism between the revolving lock 5 and the hub 7 and the radial recesses on second horizontal flange of hub 7. It is noted that these additional features could also be implemented in the second embodiment.

With respect to the views depicted in Figs. 10 and 11 it can be seen that the configuration of the second embodiment of the connector assembly 1 according to the invention is very compact and easy to handle. The longitudinal configuration of connector element 3, however, is not mandatory and could be replaced by an elbow configuration such as in the first embodiment or by a configuration with a very slight bend. It has already been mentioned that the tubular channel 22 of hub 7 of the second embodiment is relatively short which means that, as can be seen in Fig. 11, second end 11 of connector element 3 protrudes beyond the lower surface of hub 7 so that it is the second end 11 of connector element 3 which is directly connected with a base of a catheter device.

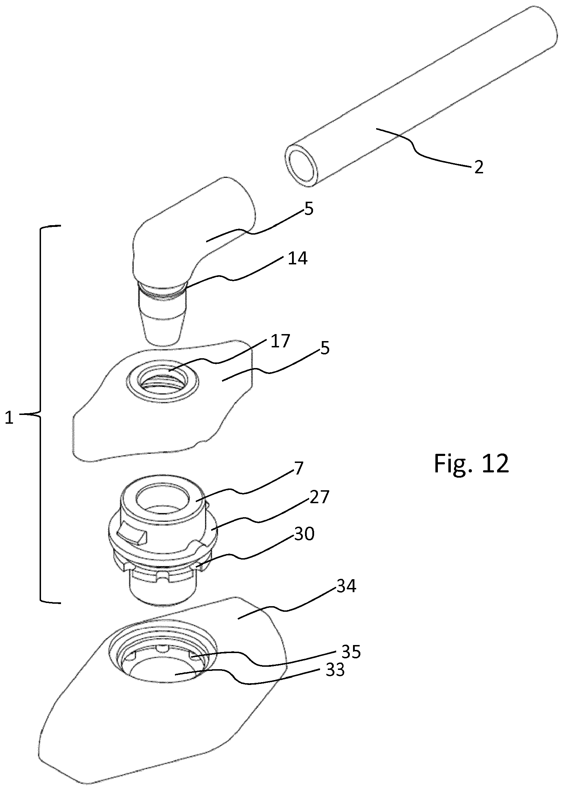

Fig. 12 shows a perspective exploded view of a connector assembly according to the first embodiment and a base of a gastrostomy device. The three components connector element 3, revolving lock 5 and hub 7 have already been described above with regard to Figs. 1 to 7. The fourth component shown in Fig. 12 is base 34 having a circular aperture 33. Semicircular protrusions 35 are formed inside aperture 33 which are configured to engage with recesses 30 on the lower second flange 29 of hub 7. The material of base 34 is usually an elastomeric material such as silicone rubber or the like whereas the hub 7 is made of a harder thermoplastic material. This means that by pressing hub 7 into the circular aperture 33 of base 34 the softer silicone rubber material will yield so that a matching fit is achieved wherein, for example, recesses 30 engage with corresponding protrusions 35 or the flanges 27, 29 engage with respective grooves on the side surface of circular aperture 33. It is essential that hub 7 is in tight engagement with the silicone rubber base 34.

Base 34 may comprise a valve-like membrane with slits which ensures a seal when the connector assembly is not connected with base 34 and which is penetrated by the lower end of connector element 3 or hub 7 when the connector assembly is fully attached to the base. Such anti-reflux valve or membrane may be built-in into the hub 7. The tapered tube portion 15 of the connector 3 serves for easy engagement with that anti-reflux valve and inner lumen surface of the base 34 or the hub 7.

Silicon rubber is a well-known material for medical applications. It is relatively cheap and easy to manufacture, comprises resilient characteristics among other characteristics which are beneficial in the medical field such as low toxicity, thermal stability, does not support microbiological growth etc. It should be noted that other suitable materials may be used for the base such as silicon-based deformable materials, thermoplastic elastomer material (TPE) or the like. Advantageously, the material of the components of the connector assembly is formed of relatively hard thermoplastic polymer material such as polypropylene (PP), polyvinylchloride (PVC) and polyethylene (PE). Other types of material are also possible. The technology for producing the components can be injection molding or other molding techniques but also 3D printing.

With the subject matter of the present invention a connector assembly has been provided which comprises a simple design, is easy to manufacture and ensures an enhanced performance and usability.

## Claims

1. Connector assembly (1) for coupling a tube (2) to a base (34) of a catheter device, the connector assembly (1) comprising:
a connector element (3) having a channel (6) and being configured to be coupled at a first end (9) to the tube (2),
a revolving lock (5) having an opening (17) for accommodating the connector element (3) and being configured to engage with the connector element (3) thereby enabling a free rotation around a rotation axis (4),
a hub (7) having a tubular channel (22) and being configured to be attached to the base (34),
wherein a second end (11) of the connector element (3) is configured to be at least partially inserted into the tubular channel (22) of the hub (7),
wherein the revolving lock (5) is configured to be releasably locked with the hub (7), and
wherein the rotation of the connector element (3) is configured to be decoupled from the locking mechanism between the revolving lock (5) and the hub (7),
**characterized in that**
the locking mechanism between the revolving lock (5) and the hub (7) comprises at least one thread element (31, 20) on an outer surface (25) of the hub (7) and on an inner surface of the revolving lock (5).

2. Connector assembly (1) for coupling a tube (2) to a base (34) of a catheter device, the connector assembly (1) comprising:
a connector element (3) having a channel (6) and being configured to be coupled at a first end (9) to the tube (2),
a revolving lock (5) having an opening (17) for accommodating the connector element (3) and being configured to engage with the connector element (3) thereby enabling a free rotation around a rotation axis (4),
a hub (7) having a tubular channel (22) and being configured to be attached to the base (34),
wherein a second end (11) of the connector element (3) is configured to be at least partially inserted into the tubular channel (22) of the hub (7),
wherein the revolving lock (5) is configured to be releasably locked with the hub (7), and
wherein the rotation of the connector element (3) is configured to be decoupled from the locking mechanism between the revolving lock (5) and the hub (7),
**characterized in that**
the locking mechanism between the revolving lock (5) and the hub (7) comprises at least one thread element (31a, 20a) on an inner surface of the hub (7) and on an outer surface of the revolving lock (5).

3. Connector assembly (1) according to claim 1, **characterized in that** the locking mechanism comprises a male thread portion (31) on an outer surface (25) of the hub (7) in cooperation with a female thread portion (20) on an inner surface of the revolving lock (5).

4. Connector assembly (1) according to claim 2, **characterized in that** the locking mechanism comprises a female thread portion (31a) on an inner surface of the hub (7) in cooperation with a male thread portion (20a) on an outer surface of the revolving lock (5).

5. Connector assembly (1) according to any of the previous claims, **characterized in that** the locking mechanism comprises at least one protrusion (32) extending from a circumferential surface of the hub (7) in cooperation with at least one recess (18) arranged on a circumferential surface of the revolving lock (5).

6. Connector assembly (1) according to any of claims 1 to 4, **characterized in that** the locking mechanism comprises at least one protrusion extending from a circumferential surface of the revolving lock (5) in cooperation with at least one recess arranged on a circumferential surface of the hub (7).

7. Connector assembly (1) according to any of the previous claims, **characterized in that** the revolving lock (5) comprises at least one wing element (19) aligned in a plane perpendicular to the rotation axis (4).

8. Connector assembly (1) according to any of the previous claims, **characterized in that** the connector element (3) comprises an elbow shape wherein the first end (9) is arranged substantially perpendicular to the second end (11).

9. Connector assembly (1) according to any of the previous claims, **characterized in that** the revolving lock (5) comprises at least one grip element (24) along a circumferential surface.

10. Connector assembly (1) according to any of the previous claims, **characterized in that** the hub (7) comprises interlocking means (30) configured to engage with a corresponding means on the base.

11. Connector assembly (1) according to any of the previous claims, **characterized in that** the connector element (3) and the revolving lock (5) are preassembled as an integral component.

12. Connector assembly (1) according to claim 11, **characterized in that** the second end (9) of the connector element (3) comprises at least one groove (14) configured to engage with one or more corresponding protrusions in the opening of the revolving lock (5) to form a permanent assembly enabling the free rotation of the connector element (3) with respect to the revolving lock (5).

13. Connector assembly (1) according to any of the previous claims, **characterized in that** the second end (11) of the connector element (3) extends beyond the tubular channel (22) of the hub (7).

14. Catheter assembly comprising a tube (2), a connector assembly (1) according to any of the previous claims and catheter device having a base (34).

## Patentansprüche

1. Verbinderanordnung zur Kopplung eines Schlauchs (2) an einen Grundkörper (34) einer Kathetervorrichtung, wobei die Verbinderanordnung (1) aufweist:
ein Verbindungselement (3), das einen Kanal (6) aufweist und das dazu eingerichtet ist, an einem ersten Ende (9) an den Schlauch (2) gekoppelt zu werden,
einen Drehverschluss (5), der eine Öffnung (17) zur Aufnahme des Verbindungselements (3) aufweist und der dazu eingerichtet ist, mit dem Verbindungselement (3) einzugreifen, wodurch eine freie Drehung um eine Drehachse (4) ermöglicht wird,
ein Drehelement (7), das einen flachen Kanal (22) aufweist und das dazu eingerichtet ist, an dem Grundkörper (34) angebracht zu werden,
wobei ein zweites Ende (11) des Verbindungselements (3) dazu eingerichtet ist, mindestens teilweise in den flachen Kanal (22) des Drehelements (7) eingeführt zu werden,
wobei der Drehverschluss (5) dazu eingerichtet ist, lösbar mit dem Drehelement (7) verriegelt zu sein, und
wobei die Drehung des Verbindungselements (3) derart eingerichtet ist, dass sie den Verriegelungsmechanismus zwischen dem Drehverschluss (5) und dem Drehelement (7) entkoppelt,
**dadurch gekennzeichnet, dass**
der Verriegelungsmechanismus zwischen dem Drehverschluss (5) und dem Drehelement (7) mindestens ein Gewindeelement (31, 20) auf einer äußeren Fläche (25) des Drehelements (7) und auf einer inneren Fläche des Drehverschlusses (5) aufweist.

2. Verbinderanordnung (1) zur Kopplung eines Schlauchs (2) an einen Grundkörper (34) einer Kathetervorrichtung, wobei die Verbinderanordnung (1) aufweist:
ein Verbindungselement (3), das einen Kanal (6) aufweist und dazu eingerichtet ist, an einem ersten Ende (9) an den Schlauch (2) gekoppelt zu werden,
einen Drehverschluss (5), der eine Öffnung (17) zur Aufnahme des Verbindungselements (3) aufweist und der dazu eingerichtet ist, mit dem Verbindungselement (3) einzugreifen, wodurch eine freie Drehung um eine Drehachse (4) ermöglicht wird,
ein Drehelement (7), das einen flachen Kanal (22) aufweist und das dazu eingerichtet ist, an dem Grundkörper (34) angebracht zu werden,
wobei ein zweites Ende (11) des Verbindungselements (3) dazu eingerichtet ist, mindestens teilweise in den flachen Kanal (22) des Drehelements (7) eingeführt zu werden,
wobei der Drehverschluss (5) dazu eingerichtet ist, lösbar mit dem Drehelement (7) verriegelt zu sein, und
wobei die Drehung des Verbindungselements (3) derart eingerichtet ist, dass sie den Verriegelungsmechanismus zwischen dem Drehverschluss (5) und dem Drehelement (7) zu entkoppeln,
**dadurch gekennzeichnet, dass**
der Verriegelungsmechanismus zwischen dem Drehverschluss (5) und dem Drehelement (7) mindestens ein Gewindeelement (31a, 20a) auf einer inneren Fläche (25) des Drehelements (7) und auf einer äußeren Fläche des Drehverschlusses (5) aufweist.

3. Verbinderanordnung (1) nach Anspruch 1, **dadurch gekennzeichnet, dass** der Verriegelungsmechanismus einen Außengewindeabschnitt (31) an einer äußeren Fläche (25) des Drehelements (7) aufweist, der mit einem Innengewindeabschnitt (20) auf einer inneren Fläche des Drehverschlusses (5) zusammenwirkt.

4. Verbinderanordnung (1) nach Anspruch 2, **dadurch gekennzeichnet, dass** der Verriegelungsmechanismus einen Innengewindeabschnitt (31a) auf einer inneren Fläche des Drehelements (7) aufweist, der mit einem Außengewindeabschnitt (20a) auf einer äußeren Fläche des Drehverschlusses (5) zusammenwirkt.

5. Verbinderanordnung (1) nach einem der vorhergehenden Ansprüche, **dadurch gekennzeichnet, dass** der Verriegelungsmechanismus mindestens einen Vorsprung (32) aufweist, der sich von einer Umfangsfläche des Drehelements (7) erstreckt, der mit mindestens einer Ausnehmung (18) zusammenwirkt, die auf einer Umfangsfläche des Drehverschlusses (5) angeordnet ist.

6. Verbinderanordnung (1) nach einem der Ansprüche 1 bis 4, **dadurch gekennzeichnet, dass** der Verriegelungsmechanismus mindestens einen Vorsprung umfasst, der sich von einer Umfangsfläche des Drehverschlusses (5) aus erstreckt, der mit mindestens einer Ausnehmung zusammenwirkt, die auf einer Umfangsfläche des Drehelements (7) angeordnet ist.

7. Verbinderanordnung (1) nach einem der vorhergehenden Ansprüche, **dadurch gekennzeichnet, dass** der Verriegelungsmechanismus (5) mindestens ein Flügelelement (19) aufweist, das axial in einer Ebene ausgerichtet ist, die senkrecht zur Drehachse (4) ist.

8. Verbinderanordnung (1) nach einem der vorhergehenden Ansprüche, **dadurch gekennzeichnet, dass** das Verbindungselement (3) eine Ellbogenform aufweist, wobei das erste Ende (9) im Wesentlichen senkrecht zu dem zweiten Ende (11) angeordnet ist.

9. Verbinderanordnung (1) nach einem der vorhergehenden Ansprüche, **dadurch gekennzeichnet, dass** der Drehverschluss (5) mindestes ein Griffelement (24) entlang einer Umfangsfläche aufweist.

10. Verbinderanordnung (1) nach einem der vorhergehenden Ansprüche, **dadurch gekennzeichnet, dass** das Drehelement (7) ein Verriegelungsmittel (30) aufweist, das dazu eingerichtet ist, mit einem zugehörigen Mittel auf dem Grundkörper einzugreifen.

11. Verbinderanordnung (1) nach einem der vorhergehenden Ansprüche, **dadurch gekennzeichnet, dass** das Verbindungselement (3) und der Drehverschluss (5) als ein integrales Bauteil vormontiert sind.

12. Verbinderanordnung (1) nach Anspruch 11, **dadurch gekennzeichnet, dass** das zweite Ende (9) des Verbindungselements (3) mindestens eine Nut (14) aufweist, die dazu eingerichtet ist, mit einem oder mehreren zugehörigen Vorsprüngen in der Öffnung des Drehverschlusses (5) einzugreifen, um eine feste Anordnung zu bilden, die die freie Drehung des Verbindungselements (3) bezüglich des Drehverschlusses (5) ermöglicht.

13. Verbinderanordnung (1) nach einem der vorhergehenden Ansprüche, **dadurch gekennzeichnet, dass** das zweite Ende (11) des Verbindungselements (3) sich über den flachen Kanal (22) des Drehelements (7) hinaus erstreckt.

14. Katheteranordnung, die einen Schlauch (2), eine Verbinderanordnung (1) nach einem der vorhergehenden Ansprüche und eine Kathetervorrichtung mit einem Grundkörper (34) aufweist.

## Revendications

1. Ensemble raccord (1) pour coupler un tube (2) à une base (34) d'un dispositif cathéter,
l'ensemble raccord (1) comprenant :
un élément de raccord (3) ayant un canal (6) et étant configuré pour être couplé au niveau d'une première extrémité (9) au tube (2),
un verrou tournant (5) ayant une ouverture (17) pour accueillir l'élément de raccord (3) et étant configuré pour s'engager avec l'élément de raccord (3), permettant ainsi une rotation libre autour d'un axe de rotation (4), un moyeu (7) ayant un canal tubulaire (22) et étant configuré pour être fixé à la base (34),
dans lequel une deuxième extrémité (11) de l'élément de raccord (3) est configurée pour être insérée au moins partiellement dans le canal tubulaire (22) du moyeu (7), dans lequel le verrou tournant (5) est configuré pour être verrouillé de façon libérable au moyeu (7), et
dans lequel la rotation de l'élément de raccord (3) est configurée pour être découplée du mécanisme de verrouillage entre le verrou tournant (5) et le hub (7),
**caractérisé en ce que**
le mécanisme de verrouillage entre le verrou tournant (5) et le moyeu (7) comprend au moins un élément de filet (31, 20) sur une surface externe (25) de l'embout femelle (7) et sur une surface interne du verrou tournant (5).

2. Ensemble raccord (1) pour coupler un tube (2) à une base (34) d'un dispositif cathéter,
l'ensemble raccord (1) comprenant :
un élément de raccord (3) ayant un canal (6) et étant configuré pour être couplé au niveau d'une première extrémité (9) au tube (2),
un verrou tournant (5) ayant une ouverture (17) pour accueillir l'élément de raccord (3) et étant configuré pour s'engager avec l'élément de raccord (3) permettant ainsi une rotation libre autour d'un axe de rotation (4), un moyeu (7) ayant un canal tubulaire (22) et étant configuré pour être fixé à la base (34),
dans lequel une deuxième extrémité (11) de l'élément de raccord (3) est configurée pour être insérée au moins partiellement dans le canal tubulaire (22) du moyeu (7), dans lequel le verrou tournant (5) est configuré pour être verrouillé de façon libérable au moyeu (7), et
dans lequel la rotation de l'élément de raccord (3) est configurée pour être découplée du mécanisme de verrouillage entre le verrou tournant (5) et le moyeu (7), **caractérisé en ce que**
le mécanisme de verrouillage entre le verrou tournant (5) et le moyeu (7) comprend au moins un élément de filet (31a, 20a) sur une surface interne de l'embout femelle (7) et sur une surface externe du verrou tournant (5).

3. Ensemble raccord (1) selon la revendication 1, **caractérisé en ce que** le mécanisme de verrouillage comprend une portion de filet mâle (31) sur une surface externe (25) du moyeu (7) en coopération avec une portion de filet femelle (20) sur une surface interne du verrou tournant (5).

4. Ensemble raccord (1) selon la revendication 2, **caractérisé en ce que** le mécanisme de verrouillage comprend une portion de filet femelle (31a) sur une surface interne du moyeu (7) en coopération avec une portion de filet mâle (20a) sur une surface externe du verrou tournant (5).

5. Ensemble raccord (1) selon l'une quelconque des revendications précédentes, **caractérisé en ce que** le mécanisme de verrouillage comprend au moins une protubérance (32) s'étendant depuis une surface circonférentielle de le moyeu (7) en coopération avec au moins un évidement (18) agencé sur une surface circonférentielle du verrou tournant (5).

6. Ensemble raccord (1) selon l'une quelconque des revendications 1 à 4, **caractérisé en ce que** le mécanisme de verrouillage comprend au moins une protubérance s'étendant depuis une surface circonférentielle du verrou tournant (5) en coopération avec au moins un évidement agencé sur une surface circonférentielle du moyeu (7).

7. Ensemble raccord (1) selon l'une quelconque des revendications précédentes, **caractérisé en ce que** le verrou tournant (5) comprend au moins un élément d'ailette (19) aligné dans un plan perpendiculaire à l'axe de rotation (4).

8. Ensemble raccord (1) selon l'une quelconque des revendications précédentes, **caractérisé en ce que** l'élément de raccord (3) comprend une forme de coude dans laquelle une première extrémité (9) est agencée sensiblement perpendiculaire à la deuxième extrémité (11).

9. Ensemble raccord (1) selon l'une quelconque des revendications précédentes, **caractérisé en ce que** le verrou tournant (5) comprend au moins un élément de préhension (24) le long d'une surface circonférentielle.

10. Ensemble raccord (1) selon l'une quelconque des revendications précédentes, **caractérisé en ce que** le moyeu (7) comprend des moyens d'enclenchement (30) configurés pour s'engager avec un moyen correspondant sur la base.

11. Ensemble raccord (1) selon l'une quelconque des revendications précédentes, **caractérisé en ce que** l'élément de raccord (3) et le verrou tournant (5) sont préassemblés sous forme de composant solidaire.

12. Ensemble raccord (1) selon la revendication 11, **caractérisé en ce que** la deuxième extrémité (9) de l'élément de raccord (3) comprend au moins une rainure (14) configurée pour s'engager avec une ou plusieurs protubérances correspondantes dans l'ouverture du verrou tournant (5) pour former un ensemble permanent permettant la rotation libre de l'élément de raccord (3) par rapport au verrou tournant (5).

13. Ensemble raccord (1) selon l'une quelconque des revendications précédentes, **caractérisé en ce que** la deuxième extrémité (11) de l'élément de raccord (3) s'étend au-delà du canal tubulaire (22) du moyeu (7).

14. Ensemble cathéter comprenant un tube (2), un ensemble raccord (1) selon l'une quelconque des revendications précédentes et un dispositif cathéter ayant une base (34).
